(19) Europäisches Patentamt / European Patent Office / Office européen des brevets

(11) **EP 3 064 060 A1**

(12) **EUROPEAN PATENT APPLICATION**
published in accordance with Art. 153(4) EPC

(43) Date of publication:
**07.09.2016 Bulletin 2016/36**

(51) Int Cl.:
*A01K 67/00* (2006.01)    *A01K 15/00* (2006.01)

(21) Application number: **13896660.1**

(22) Date of filing: **30.10.2013**

(86) International application number:
**PCT/JP2013/079462**

(87) International publication number:
**WO 2015/063900 (07.05.2015 Gazette 2015/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fujitsu Limited**
**Kawasaki-shi, Kanagawa 211-8588 (JP)**

(72) Inventors:
- **YOKOO, Kaoru**
  **Kawasaki-shi**
  **Kanagawa 211-8588 (JP)**
- **NISHIDOI, Takeshi**
  **Kawasaki-shi**
  **Kanagawa 211-8588 (JP)**

- **SUGIYAMA, Jun**
  **Kawasaki-shi**
  **Kanagawa 211-8588 (JP)**
- **TAKAGI, Junichi**
  **Kawasaki-shi**
  **Kanagawa 211-8588 (JP)**
- **IKENOUCHI, Takayuki**
  **Yokohama-shi**
  **Kanagawa 2200012 (JP)**
- **URABE, Hiroo**
  **Yokohama-shi**
  **Kanagawa 2200012 (JP)**

(74) Representative: **Schultes, Stephan**
**Haseltine Lake LLP**
**300 High Holborn**
**London WC1V 7JH (GB)**

(54) **BIOLOGICAL SENSING SYSTEM, BIOLOGICAL SENSING METHOD, AND BIOLOGICAL SENSING PROGRAM**

(57) The number of errors, caused by a displacement of a biological sensor from a placement position, of sensing results is reduced. Biological information detected by a biological information detection unit that is placed in a specified portion of a living thing and is configured to detect the biological information of the living thing is obtained, the amount of a displacement detected by a position displacement detection unit that detects the amount of the displacement of the position of the biological information detection unit from the specified position when the biological information is detected is obtained, and optimum biological information is estimated from the obtained biological information in accordance with the amount of the displacement.

F I G. 1

EP 3 064 060 A1

## Description

Technical Field

The present invention relates to biological sensing.

Background Art

Use of a nose ring that is less apt to drop while maintaining contact with a nasal septum within a living body in a stage prior to implantation is promising biological sensing means for non-invasively managing pastured livestock. A biological sensing technology of livestock using such a nose ring is referred to as nose ring sensing.

In the meantime, in the case of temperature sensing for measuring the temperature of livestock, accuracy in detecting a temperature change as small as 0.1°C is demanded.

Prior Art Document

Patent Document

Patent Document 1: Japanese National Publication of International Patent Application No. 2002-541866

Summary of the Invention

Problems to be Solved by the Invention

In nose ring sensing, an error occurs in sensing results due to a displacement of a biological sensor from the nasal septum.

Accordingly, in one aspect, an object of the present invention is to reduce errors in sensing results caused by a displacement of a biological sensor from a placement position.

Means for Solving the Problems

A biological sensing system in one aspect includes a biological information detection unit, a position displacement detection unit, and an estimation unit. The biological information detection unit is placed in a specified portion of a living thing, and detects biological information of the living thing. The position displacement detection unit detects the amount of a displacement of the biological information detection unit from the specified portion when the biological information is detected. The estimation unit estimates optimum biological information from the biological information detected by the biological information detection unit in accordance with the amount of the displacement.

Effects of the Invention

With a biological sensing system according to an embodiment, errors in sensing results caused by a displacement of a biological sensor from a placement position can be reduced.

Brief Description of Drawings

FIG. 1 illustrates an example of a block diagram of a biological sensing system;
FIG. 2 illustrates an example of a biological sensing system according to a first embodiment;
FIG. 3 illustrates an example of the shape of a nose ring.
FIG. 4 illustrates a structure of a sensor data table according to the first embodiment;
FIG. 5 is explanatory diagrams of a method for calculating a ring inclination value from inclination sensor data;
FIG. 6 is an explanatory diagram for obtaining the position of a sensor;
FIG. 7 illustrates an example of a lookup table according to the first embodiment;
FIG. 8 is a flowchart illustrating operations of a nose ring sensing system according to the first embodiment;
FIG. 9 illustrates a modification example of a biological sensing system;
FIG. 10 illustrates an example of a configuration of a biological sensing system according to a second embodiment;
FIG. 11 illustrates a structure of a sensor data table according to the second embodiment;
FIG. 12 illustrates an example of a lookup table according to the second embodiment;
FIG. 13 illustrates an example of a configuration of a biological sensing system according to a third embodiment;
FIG. 14 illustrates an example of a nose ring including a plurality of biological sensors;
FIG. 15 illustrates a structure of a sensor data table according to the third embodiment;
FIG. 16 is an explanatory diagram for selecting a biological sensor closest to the nasal septum from among the plurality of biological sensors;
FIG. 17 is a flowchart illustrating operations of a nose ring sensing system according to a fourth embodiment;
FIG. 18 is an explanatory diagram of an occurrence of erroneous detection or erroneous warning when livestock lies down;
FIG. 19 illustrates an example of a configuration of a biological sensing system according to a fifth embodiment;
FIG. 20 illustrates an example of a lookup table according to the fifth embodiment;

FIG. 21 is a flowchart illustrating operations of a nose ring sensing system according to the fifth embodiment; and

FIG. 22 illustrates an example of a hardware configuration of a server and a display device;

Best Mode for Carrying Out the Invention

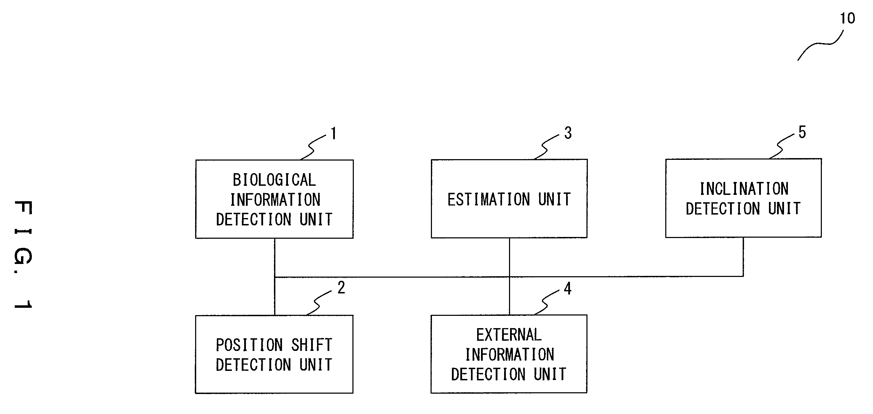

FIG. 1 illustrates an example of a functional block diagram of a biological sensing system according to an embodiment. The biological sensing system 10 includes a biological information detection unit 1, a position displacement detection unit 2, an estimation unit 3, an external information detection unit 4, and an inclination detection unit 5.

The biological information detection unit 1 is placed in a first portion of a living thing, and detects biological information of the living thing.

The position displacement detection unit 2 detects the amount of a displacement of the position of the biological information detection unit from the first portion when the biological information is detected. Moreover, the position displacement detection unit 2 detects a change in the acceleration of gravity, and detects the amount of a displacement on the basis of the change in the acceleration of gravity.

The estimation unit 3 estimates optimum biological information in accordance with the amount of a displacement from the biological information detected by the biological information detection unit. Moreover, the estimation unit 3 selects or abandons biological information in accordance with the amount of a displacement, and estimates the selected biological information as optimum biological information. Moreover, when the amount of a displacement is equal to or smaller than a specified threshold value, the estimation unit 3 estimates biological information as optimum biological information. Additionally, the estimation unit 3 corrects biological information in accordance with the amount of a displacement, and estimates the corrected biological information as optimum biological information. Furthermore, the estimation unit 3 selects one piece of biological information in accordance with the amount of a displacement from among a plurality of pieces of biological information detected by a plurality of biological information detection units 1, and estimates the selected biological information as optimum biological information. Still further, the estimation unit 3 corrects the selected piece of biological information in accordance with the amount of a displacement, and estimates the corrected biological information as optimum biological information. Still further, the estimation unit 3 estimates optimum biological information on the basis of a plurality of pieces of biological information detected by the plurality of biological information detection units 1, and the amount of a displacement. Still further, the estimation unit 3 corrects the biological information detected by the biological information detection unit 1 in accordance with external information detected by the external information detection unit 4, and the amount of a displacement, and estimates the corrected biological information as optimum biological information. Still further, the estimation unit 3 estimates biological information of a living thing in accordance with external information detected by the external information detection unit 4, the amount of a displacement, and a plurality of pieces of biological information detected by the plurality of biological information detection units 1. Still further, the estimation unit 3 corrects the amount of a displacement on the basis of an inclination of a second portion, detected by the inclination detection unit 5, in a gravitational direction. Still further, the estimation unit 3 corrects the biological information detected by the biological information detection unit 1 in accordance with the type, the sex, the age, the nose size, and the nasal septum thickness of a living thing.

The external information detection unit 4 detects external information about the periphery of a living thing when biological information is detected.

The inclination detection unit 5 is placed in the second portion of the living thing, and detects the inclination of the second portion in the gravitational direction.

(First embodiment)

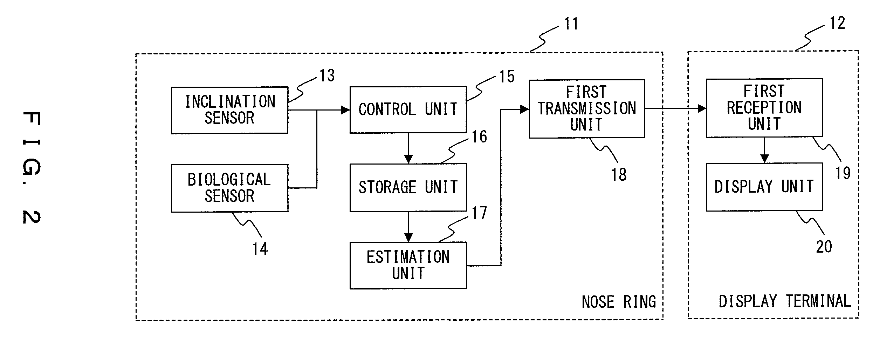

An example of a system configuration of this embodiment is described. FIG. 2 illustrates an example of a configuration of a nose ring sensing system according to this embodiment.

As illustrated in FIG. 2, the nose ring sensing system includes a nose ring 11 that performs sensing, and a display terminal 12 that displays a result of the sensing. The nose ring 11 includes an inclination sensor 13, a biological sensor 14, a control unit 15, a storage unit 16, an estimation unit 17, and a first transmission unit 18. Moreover, the display terminal 12 includes a first reception unit 19 and a display unit 20. The inclination sensor 13 and the control unit 15 are an example of the position displacement detection unit 2. The biological sensor 14 is one example of the biological information detection unit 1. The estimation unit 17 is one example of the estimation unit 3.

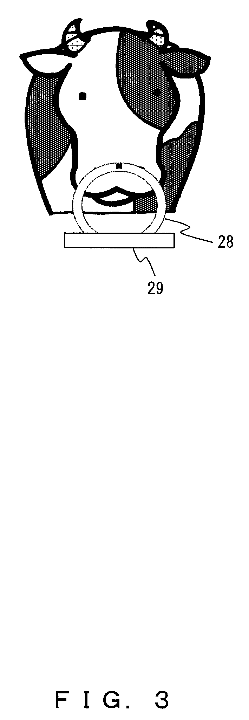

FIG. 3 illustrates one example of the shape of the nose ring. The nose ring 11 includes a ring part 28, and a block part 29, and is attached, for example, to the nose of a cow as illustrated in FIG. 3.

The inclination sensor 13 is used to detect the inclination of the nose ring 11. In this embodiment, the inclination sensor 13 is, for example, a two-axis acceleration sensor. The two-axis acceleration sensor is incorporated into the block part 29 of the nose ring 11 so that two axes can become parallel to the surface of the ring. Here, a sensor for detecting a distance to the nasal septum may be used as the inclination sensor 13. The inclination sensor 13 is not limited to this one.

The biological sensor 14 measures biological data of a living thing to which the nose ring 11 is attached. In the first embodiment, the biological sensor 14 is a temperature sensor for measuring the temperature of a living thing to which the nose ring 11 is attached. The temperature sensor is buried in the central portion of the ring part 28 of the nose ring 11.

Here, data measured by the inclination sensor 13 and that measured by the biological sensor 14 are respectively referred to as inclination sensor data and biological data in the following explanation. Moreover, in the following explanation of this embodiment, both the data measured by the inclination sensor 13 and that measured by the biological sensor 14 are referred to simply as sensor data in some cases.

The control unit 15 periodically obtains sensor data for a certain time period from the inclination sensor 13 and the biological sensor 14.

The control unit 15 obtains biological data in an analog format from the biological sensor 14. Then, the control unit 15 performs an analog-to-digital conversion for the obtained analog value, and stores the converted digital value in the storage unit 16.

Additionally, the control unit 15 obtains, from the inclination sensor 13, inclination sensor data that is measured at the same time as the measurement time of the biological data obtained from the biological sensor 14. The timing at which the control unit 15 obtains the sensor data is synchronized with that at which the control unit 15 obtains biological data from the biological sensor 14. The obtained inclination sensor data has an analog format. Then, the control unit 15 performs an A/D conversion for the analog value of the inclination sensor data, and stores the digital value of the result of the conversion in the storage unit 16, similarly to the biological data.

Note that sensor data are respectively measured when the control unit 15 obtains the sensor data from the biological sensor 14 and the inclination sensor 13. The control unit 15 simultaneously obtains the sensor data of the biological sensor 14 and that of the inclination sensor 13 in this way, so that the biological sensor data and the inclination sensor data that are measured simultaneously can be obtained. Moreover, also in the other embodiments, times at which the control unit 15 obtains sensor data respectively from the various sensors are a measurement time of the sensor data.

Here, in this embodiment, the obtained sensor data is time-series data, periodically measured for a specified time period (such as several seconds to several tens of seconds) of the temperature sensor and the two-axis acceleration sensor. The control unit 15 is connected to the biological sensor 14 via a bus through the ring part 28, and incorporated into the block part 29 of the nose ring 11.

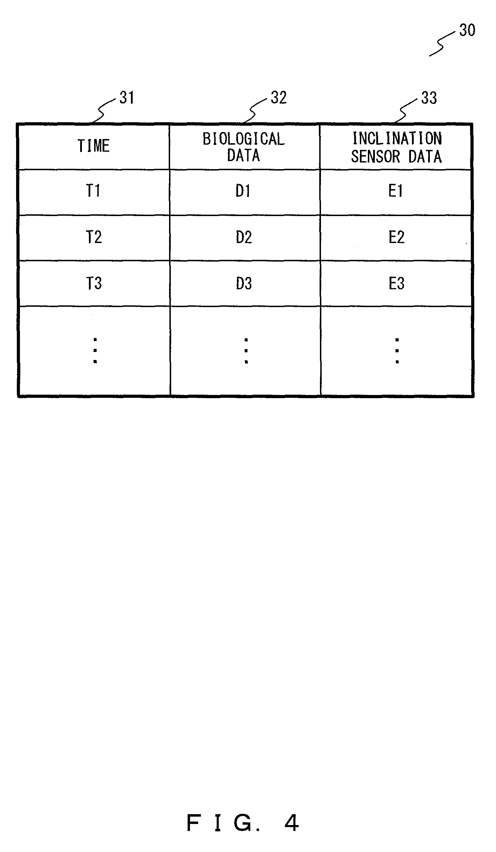

The storage unit 16 stores a sensor data table in which an association is made between sensor data and a measurement time of the sensor data. The storage unit 16 receives, from the control unit 15, sensor data, and the measurement time of the sensor data, and stores the received information in the sensor data table. Here, a structure of the sensor data table is illustrated in FIG. 4. The sensor data table 30 includes data items (columns) such as a time (or a date and time) 31, biological data 32, and inclination sensor data 33. The time 31 indicates a time at which values of corresponding biological sensor 14 and inclination sensor 13 are measured. The biological data 32 is a value of biological data measured by the biological sensor 14 at the time 31. The inclination sensor data 33 is a value of inclination sensor data measured by the inclination sensor 13 at the time 31.

Note that the sensor data table 30 is managed for each cow to which the nose ring 11 is attached. This applies to the other embodiments.

Additionally, the storage unit 16 stores data indicating a relationship between the inclination of the nose ring 11 and an influence that the inclination of the nose ring 11 exerts on biological data. Such data is referred to as correlation data in the following explanation. The correlation data is data, for example, in the form of a lookup table (LUT in the drawings), or a function expression (Func in the drawings). The correlation data is used to correct biological data in accordance with the inclination of the nose ring 11. The storage unit 16 is incorporated in the block part 29 of the nose ring 11.

The estimation unit 17 calculates a value indicating the inclination of the nose ring in the gravitational direction from the inclination sensor data 33 stored in the sensor data table 30. Here, the value indicating the inclination of the nose ring in the gravitational direction is referred to as a ring inclination value in the following explanation. When a displacement of the nose ring 11 from the nasal septum of the central portion of the nose ring 11 is large, namely, when the ring inclination value is equal to or larger than a specified value, the estimation unit 17 determines that the obtained biological data is not suitable for use. In contrast, when the ring inclination value is smaller than the specified value, the estimation unit 17 corrects the value of the biological data by using the correlation data. Then, the estimation unit 17 outputs the corrected biological data to the first transmission unit 18. Here, the biological data corrected by the estimation unit 17 is sometimes referred to as correction data in the following explanation. The estimation unit 17 is incorporated into the block part of the nose ring 11.

The first transmission unit 18 obtains the correction data from the estimation unit 17, and transmits the correction data to the first reception unit 19 via an external network. The first transmission unit 18 makes a connection to the external network. The connected external network may be, for example, a network of a cellular phone, a wireless LAN, or the like. The first transmission unit 18 is incorporated into the block part 29 of the nose ring 11.

The first reception unit 19 receives the correction data transmitted from the transmission unit, and outputs the received data to the display unit 20. The first reception unit 19 makes a connection to the first transmission unit 18 via an external network.

The display unit 20 obtains the correction data from the first reception unit 19, converts the correction data into a form that can be reproduced on the display terminal 12, and displays the data on the display terminal 12. Here, examples of the form that can be reproduced on the display terminal 12 include HTML (HyperText Markup Language) 5 and the like, which can be browsed using a tablet, a smartphone, a personal computer and the like. Moreover, when a value of correction data satisfies a prerequisite, the display unit 20 issues a warning to a predetermined terminal along with the data, or may issue an instruction to prompt the predetermined device to perform a specified operation. Alternatively, when a trend of the correction data in the past within a specified time period satisfies the prerequisite, the display unit 20 may issue a warning to a predetermined terminal along with the data, or may issue an instruction for prompting the predetermined device to perform a specified operation.

Here, the following explanation describes that biological data and inclination sensor data that are measured at the same time have an association. The following explanation also describes that biological data measured at a specified time, and the inclination of the nose ring 11 at that time, namely, a ring inclination value, have an association.

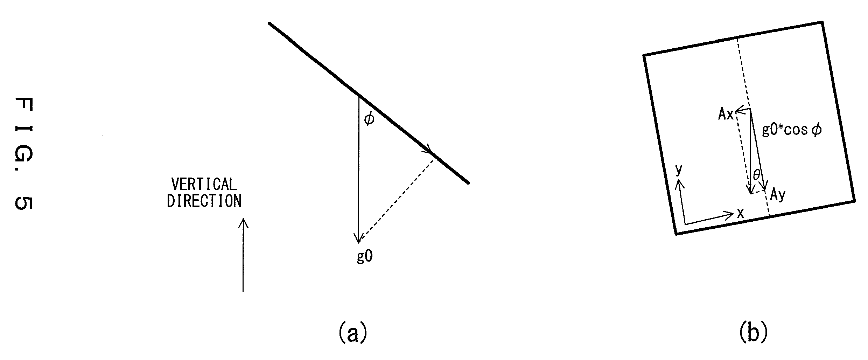

A method by which the estimation unit 17 calculates a ring inclination value from the inclination sensor data 33 is described next. FIG. 5 is explanatory diagrams of the method for calculating a ring inclination value from the inclination sensor data 33. The estimation unit 17 estimates a gravitational direction projected onto a planar surface of the ring on the basis of a ratio obtained by filtering the value of the inclination sensor data 33 through an LPF (low-pass filter), and converts the value into an inclination of the ring in the gravitational direction.

Here, the method is described by assuming that the value of the inclination sensor data measured by the two-axis acceleration sensor is (Ax,Ay). Assuming that the inclination of the surface of the nose ring in a vertical direction and the inclination within the surface of the nose ring are respectively $\varphi$ and $\theta$ (ring inclination value) as illustrated in FIG. 5 when the inclination of the nose ring 11 is considered, a projection of the acceleration of gravity on the surface of the nose ring is g0*cos $\phi$ by using the acceleration of gravity g0. Therefore, the outputs of the two-axis acceleration sensor are

$$Ax = -g0*\cos\phi*\sin\theta \quad \cdots (1)$$

$$Ay = -g0*\cos\phi*\cos\theta \quad \cdots (2)$$

Accordingly, to obtain the inclination $\theta$ within the surface of the nose ring,

$$Ax \,/\, Ay = \tan\theta$$

is acquired by dividing the expression (1) by the expression (2), except in the case of $\phi$=90 degrees. As a result, the inclination $\theta$ can be obtained as follows.

$$\theta = \mathrm{atan}(Ax/Ay)$$

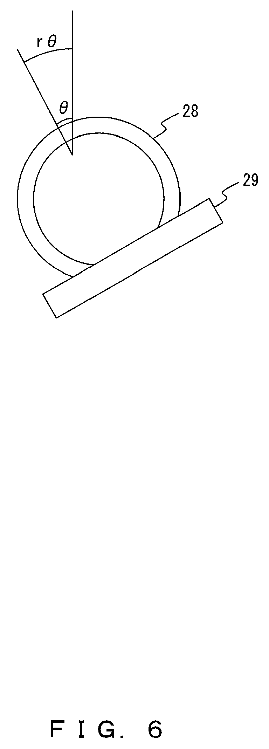

The state of $\phi$=90 degrees is not expected to occur when a cow is standing up if the placement position and the gravity of the nose ring 11 are taken into account. Therefore, this calculation is valid in almost all cases. Moreover, using a three-axis accelerometer enables the state of $\phi$=90 degrees to be detected. In such a case, the resultant value can be excluded. Moreover, the position of the sensor can be obtained from the ring inclination value $\theta$. FIG. 6 is an explanatory diagram for obtaining the position of the sensor from the ring inclination value $\theta$. Assuming that the radius of the ring is r, the position of the sensor is indicated as r$\theta$. The ring inclination value $\theta$ may be referred to as the position of the sensor r$\theta$ in the following explanation.

Next, a specific operation that the estimation unit 17 performs to determine whether a displacement of the nose ring 11 from the nasal septum is large is described. The estimation unit 17 determines whether the ring inclination value calculated on the basis of the inclination sensor data 33 is equal to or larger than a specified threshold value. When the ring inclination value is equal to or larger than the specified threshold value, the estimation unit 17 extracts a time at which the inclination sensor data 33 used to calculate the ring inclination value is measured. Then, the estimation unit 17 determines that the biological data 32 measured at the same time as the extracted time is unsuitable data.

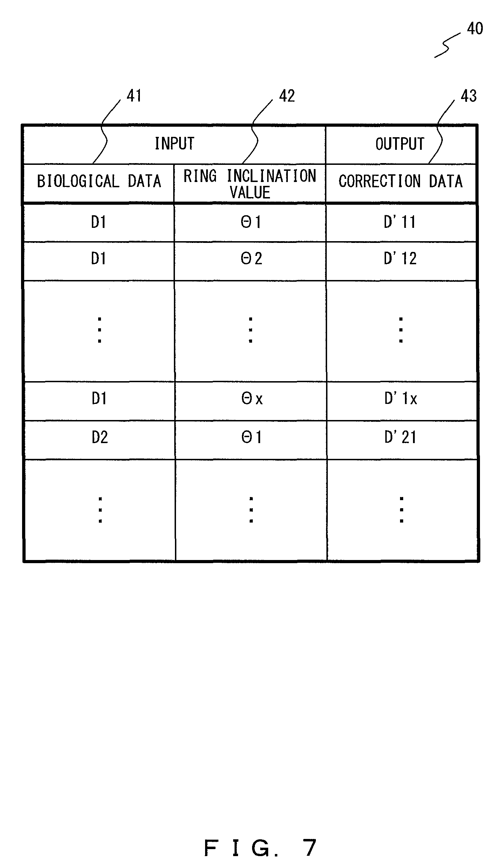

A method by which the estimation unit 17 corrects the value of biological data by using correlation data is described next. Specifically, a case where the correlation data is a lookup table is described. Here, one example of the lookup table is illustrated in FIG. 7. The lookup table 40 includes, as input information, data items such as biological data 41, and a ring inclination value 42. The lookup table 40 also includes, as output information, a data item such as correction data 43. Values of the items of the biological data 41 and the ring inclination value 42 have a specified range. As the input information, an arbitrary number or all of the combinations of the biological data 41 and the ring inclination value 42 are stored. The estimation unit 17 extracts a row of a combination of input information including biological data 32 and a ring inclination value 42 associated with the biological data 32, and obtains a value of the correction data 43 in the extracted row.

When the correlation data is data in the form of a function, the estimation unit 17 gives, to the function, the value of biological data and the ring inclination value associated with the biological data as input information, and obtains a result of the calculation of the function as correction data. Assuming that the correction data, the biological data, and the ring inclination value are respectively T', T and θ, the function expression is

$$T' = f(T, \theta)$$

Note that a correction process is not executed for biological data determined to be unsuitable data, and that the data is not output to the first transmission unit 18.

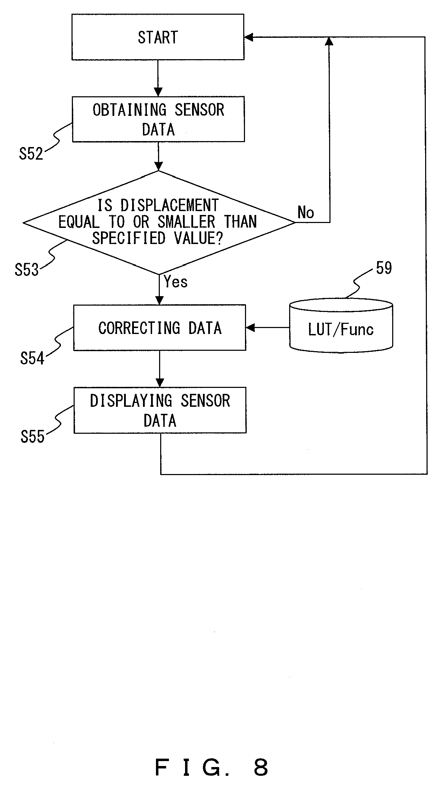

A flow of operations of the nose ring sensing system is described next. FIG. 8 is a flowchart illustrating operations of the nose ring sensing system according to the first embodiment.

Initially, the control unit 15 obtains biological data 32 from the biological sensor 14, and also obtains inclination sensor data 33 from the inclination sensor 13 (S52). Then, the control unit 15 stores, in the sensor data table 30, the obtained biological data 32 and inclination sensor data 33 in association with a measurement time.

Next, the estimation unit 17 obtains the inclination sensor data 33 from the sensor data table 30, and calculates a ring inclination value. Then, the estimation unit 17 determines whether the calculated ring inclination value is equal to or smaller than a specified threshold value (S53).

When the estimation unit 17 determines that the ring inclination value is equal to or smaller than the specified threshold value ("YES" in S53), the estimation unit 17 calculates the correction data, which is data obtained by correcting the biological data 32 associated with the inclination sensor data 33 obtained in S52 (S54). The correction data is calculated on the basis of the lookup table or the function (LUT/ Func59). Then, the estimation unit 17 outputs, to the first transmission unit 18, the correction data calculated in S54, and the corresponding time 31. Next, the first transmission unit 18 transmits the input correction data and corresponding time 31 to the first reception unit 19 of the display terminal 12.

Upon receipt of the correction time and the corresponding time 31, the first reception unit 19 outputs the received correction data and corresponding time 31 to the display unit 20. The display unit 20 converts the received correction data and corresponding time 31 into a form that can be displayed, and displays the data and the time 31 (S55) . Then, the flow proceeds to the start of the process.

When the estimation unit 17 determines that the ring inclination value is larger than the specified threshold value ("NO" in S53), the flow returns to the start of the process.

(Modification example 1)

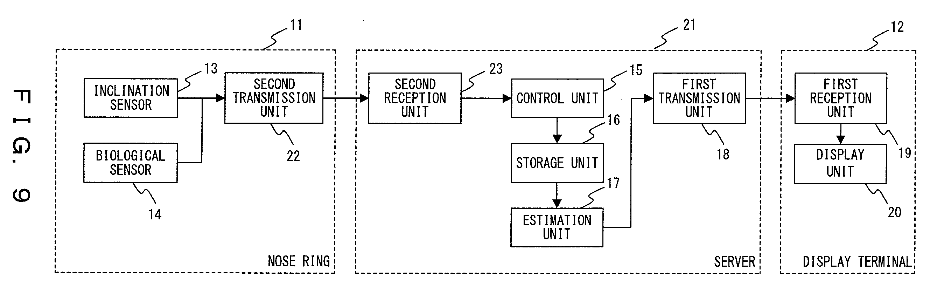

The first embodiment has been described by assuming that the control unit 15, the storage unit 16, and the estimation unit 17 are incorporated into the nose ring 11. However, these components may be incorporated into another device. A modification example of the configuration of the nose ring sensing system is illustrated in FIG. 9. As illustrated in FIG. 9, the nose ring sensing system includes the nose ring 11, a server 21, and the display terminal 12. The nose ring 11 includes the inclination sensor 13, the biological sensor 14, and a second transmission unit 22. The server 21 includes the control unit 15, the storage unit 16, the estimation unit 17, the first transmission unit 18, and a second reception unit 23. The display terminal 12 includes the first reception unit 19 and the display unit 20. The modification example is different from the first embodiment in that the control unit 15 obtains data of the biological sensor 14 and the inclination sensor 13 via a network. The second transmission unit 22 obtains the sensor data from the inclination sensor 13 and the biological sensor 14, and transmits the obtained data to the second reception unit 23 of the server 21. Upon receipt of the sensor data, the second reception unit 23 outputs the received sensor data to the control unit 15.

Another modification example may be configured by incorporating the display unit 20 of the first embodiment in the nose ring 11, or may be configured by incorporating the display unit 20 of the modification example in the server 21.

(Second embodiment)

In the first embodiment, biological data is corrected by the estimation unit 17 on the basis of a ring inclination value. In the second embodiment, in contrast, biological data is corrected on the basis of data obtained by an external sensor 24 further. Thus, errors in low-invasiveness sensing results caused by an external noise, such as an influence of an external temperature or the like in temperature sensing, can be reduced.

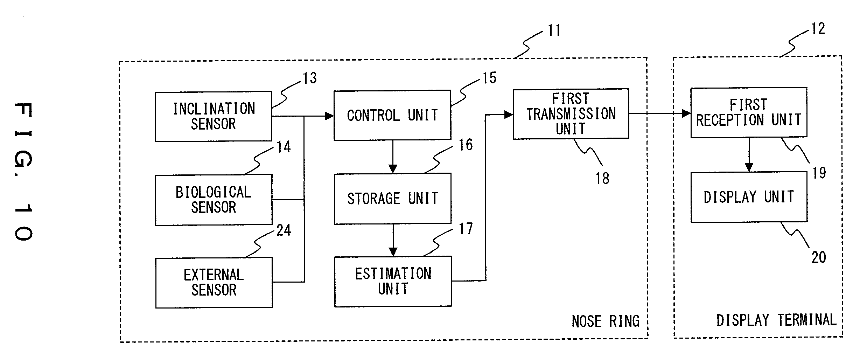

FIG. 10 illustrates a configuration of the nose ring sensing system according to the second embodiment. The configuration of the second embodiment is different from that of the first embodiment in that the nose ring 11 further includes the external sensor 24. The external sensor 24 is one example of the external information detection unit 4. The inclination sensor 13, the biological sensor 14, the first transmission unit 18, the first reception unit 19, and the display unit 20 are identical to those described in the first embodiment. Differences in the control unit 15, the storage unit 16, and the estimation unit 17 from those of the first embodiment are described below.

The external sensor 24 is, for example, a sensor for detecting an external temperature. The external sensor 24 is incorporated into the block part 29 of the nose ring 11.

The control unit 15 periodically obtains sensor data for a certain time period from the inclination sensor 13, the biological sensor 14, and the external sensor 24. The control unit 15 obtains, from the external sensor 24, data of the external sensor measured at the same time as the measurement time of the sensor data obtained from the biological sensor 14 and the inclination sensor 13. Then, the control unit 15 performs an A/D conversion for an analog value of the external sensor data, and stores, in the storage unit 16, a digital value of the result of the conversion. The sensor data obtained here is, for example, time-series data, periodically obtained for a specified time period (several seconds to several tens of seconds), of the temperature sensor, the acceleration sensor, and the external sensor 24. Note that the timings at which the control unit 15 obtains the sensor data respectively from the inclination sensor 13, the biological sensor 14, and the external sensor 24 are synchronized with one another.

Here, data measured by the external sensor 24 is referred to as external sensor data in the following explanation. In the following explanation of the second embodiment, all pieces of data measured by the inclination sensor 13, the biological sensor 14, and the external sensor 24 are referred to simply as sensor data in some cases.

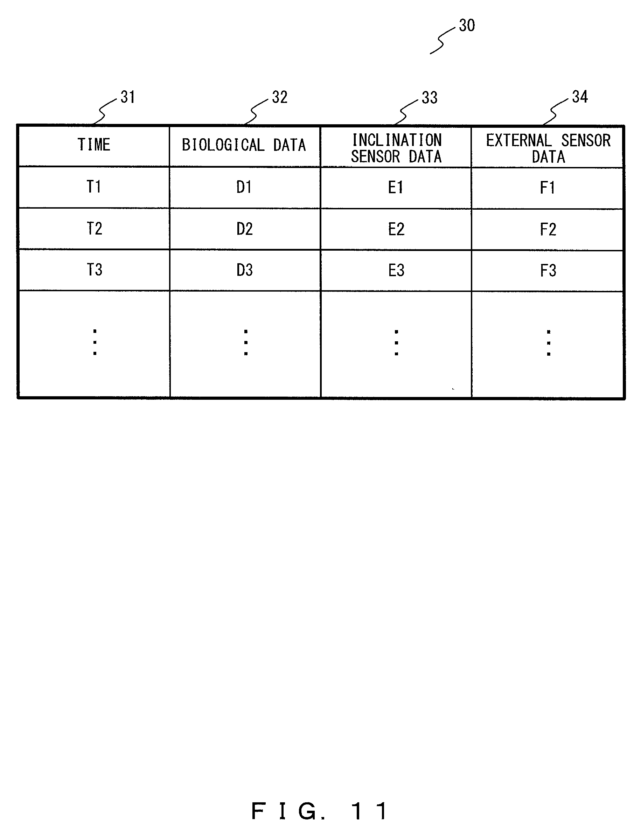

The storage unit 16 stores the sensor data table 30 in which an association is made between sensor data and a measurement time of the sensor data. Here, a structure of the sensor data table according to the second embodiment is illustrated in FIG. 11. The sensor data table 30 includes data items such as the time 31, the biological data 32, the inclination sensor data 33, and the external sensor data 34. The time 31, the biological data 32, and the inclination sensor data 33 are identical to those of the first embodiment. The external sensor data 34 is a numerical value indicated by a digital value of external sensor data measured at the time 31.

Additionally, the storage unit 16 stores the inclination of the nose ring 11, the value of external sensor data, and correlation data indicating a relationship with an influence that the inclination of the nose ring 11 and the value of external sensor data exert on biological data. The correlation data is, for example, data in the form of a lookup table 40 or a function expression. The correlation data is used to correct the biological data in accordance with the inclination of the nose ring 11 and the value of external sensor data.

The estimation unit 17 calculates a ring inclination value from the inclination sensor data 33, similarly to the first embodiment.

Then, the estimation unit 17 corrects the value of biological data on the basis of the ring inclination value and the external sensor data that are measured at the same time as the biological data. Next, the estimation unit 17 outputs the corrected biological data to the first transmission unit 18.

A method by which the estimation unit 17 corrects the value of biological data on the basis of a ring inclination value and the value of external sensor data is described next.

Similarly to the first embodiment, the estimation unit 17 initially determines whether biological data is unsuitable data by judging whether a ring inclination value is equal to or smaller than a specified threshold value.

Then, the estimation unit 17 corrects the biological data when it determines that the biological data is not unsuitable data. A case where correlation data for correcting the value of biological data has the form of the lookup table 40 is initially described.

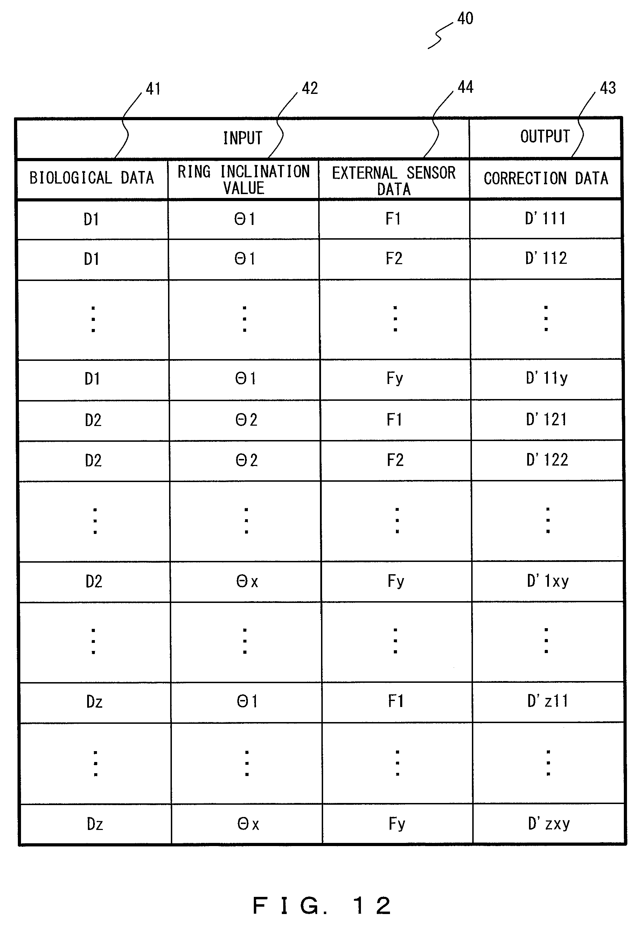

FIG. 12 illustrates one example of the lookup table 40 in the second embodiment. The lookup table 40 includes, as input information, data items such as the biological data 41, the ring inclination value 42, and the external sensor data 44. The lookup table 40 also includes, as output information, a data item such as the correction data 43. The biological data 41 and the ring inclination value 42 are identical to those of the first embodiment. Values of the entry of the external sensor data 44 have a specified range. As the input information, an arbitrary number of or all combinations of the biological data 41, the ring inclination value 42, and the external sensor data 44 are stored. The estimation unit 17 extracts a row of a combination of input information of biological data 32, and a ring inclination value 42 and external sensor data 44 that are associated with the biological data 32, and obtains the value of the correction data 43 of the extracted row.

A correction of biological data when the correlation data is data in the form of a function is described next. The estimation unit 17 gives, to a function, the value of biological data, and a ring inclination value, and external sensor data that correspond to the biological data as input information, and obtains a result of a calculation of the function as a correction value. Assuming that the correction data, the biological data, the ring inclination value, and the external sensor data are respectively T', T, θ and H, the function expression is

$$T' = f(T, \; \theta, \; H)$$

Note that the correction process is not executed for the biological data determined to be unsuitable data, and that the data is not output to the first transmission unit 18.

A flow of operations of the nose ring sensing system according to the second embodiment is described next with reference to the flowchart illustrated in FIG.8.

The control unit 15 initially obtains the biological data 32 from the biological sensor 14, also obtains the inclination sensor data 33 from the inclination sensor 13, and further obtains the external sensor data 34 from the external sensor 24 (S52). Then, the control unit 15 stores, in the sensor data table 30, the obtained biological data 32, inclination sensor data 33, and external sensor data 34 in association with a measurement time.

Next, the estimation unit 17 obtains the inclination sensor data 33 from the sensor data table 30, and calculates a ring inclination value. Next, the estimation unit 17 determines whether the calculated ring inclination value is equal to or smaller than a specified threshold value (S53).

When the estimation unit 17 determines that the ring inclination value is equal to or smaller than the specified threshold value ("YES" in S53), the estimation unit 17 calculates correction data, which is data obtained by correcting the biological data 32 associated with the inclination sensor data 33 obtained in S52 (S54). The correction data is calculated on the basis of the lookup table or the function (LUT/Func59) . Then, the estimation unit 17 outputs the correction data calculated in S54 and the associated time 31 to the first transmission unit 18. Then, the first transmission unit 18 transmits the input correction data and associated time to the first reception unit 19 of the display terminal 12.

Upon receipt of the correction data and the associated time 31, the first reception unit 19 outputs the data and the time 31 to the display unit 20. The display unit 20 converts the received correction data and associated time 31 into a form that can be displayed, and displays the data and the time (S55). Then, the flow returns to the start of the process.

When the estimation unit 17 determines that the ring inclination value is larger than the specified threshold value in S53 ("NO" in S53), the flow returns to the start of the process.

(Third embodiment)

In the third embodiment, the nose ring 11 is configured by including a plurality of biological sensors 14. In the third embodiment, the estimation unit 17 calculates correction data on the basis of a plurality of pieces of biological data obtained by the plurality of biological sensors 14, a ring inclination value, and external sensor data.

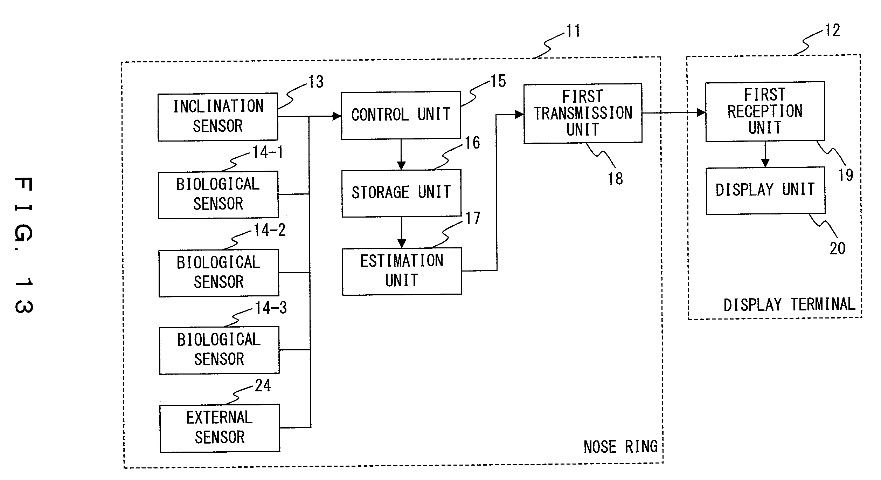

FIG. 13 illustrates a configuration of a nose ring sensing system according to a third embodiment. The configuration of the third embodiment is different from the second embodiment in that the nose ring 11 includes not one but a plurality of biological sensors 14-1, 14-2, and 14-3. The inclination sensor 13, the first transmission unit 18, the first reception unit 19, the display unit 20, and the external sensor 24 are identical to those described in the second embodiment. Moreover, differences in the control unit 15, the storage unit 16, and the estimation unit 17 from those of the first embodiment are described. Here, the biological sensors 14-1, 14-2, and 14-3 are referred to simply as the biological sensor 14 in the following explanation of the third and the fourth embodiments.

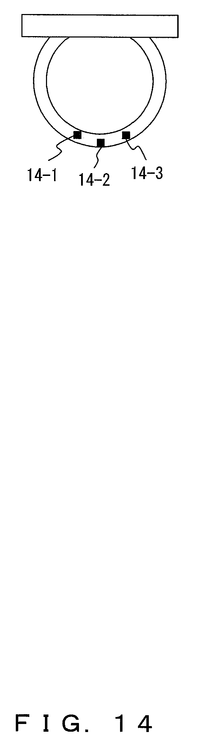

The plurality of biological sensors 14 are included in the nose ring 11. Functions of the individual biological sensors 14 are identical to those of the biological sensor 14 described in the first embodiment. Here, one example of the nose ring 11 including the plurality of biological sensors 14 is illustrated in FIG. 14. As illustrated in FIG. 14, the biological sensor 14-2 is buried in a central portion of the ring part 28 of the nose ring 11. The biological sensors 14-1 and 14-3 are buried in positions of the ring part 28 of the nose ring 11 that are symmetrical with the biological sensor 14-2. The third embodiment assumes that the number of biological sensors 14 is three, and that the positions in which the biological sensors 14-1 and 14-3 are buried are positions of the ring part 28 that are symmetrical with the biological sensor 14-2. However, the number of biological sensors 14 and the positions in which the individual biological sensors 14 are buried are not limited to the above described ones.

The control unit 15 periodically obtains sensor data for a certain time period from the inclination sensor 13, the plurality of biological sensors 14, and the external sensor 24. The control unit 15 obtains the inclination sensor data, the plurality of pieces of biological data, and the external sensor data, which are measured at the same time. Then, the control unit 15 performs an A/D conversion for analog values of the obtained sensor data, and stores digital values of the results of the conversion in the storage unit 16. The sensor data obtained here is, for example, time-series data periodically obtained for a specified time period (several seconds to several tens of seconds) of the plurality of temperature sensors, the acceleration sensor, and the external temperature sensor. Note that timings at which the control unit 15 obtains the sensor data respectively from the inclination sensor 13, the biological sensor 14, and the external sensor 24 are synchronized with one another.

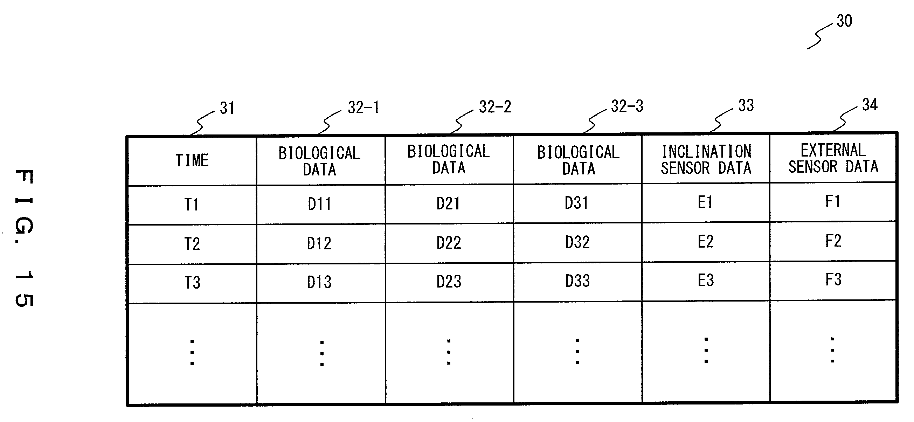

The storage unit 16 stores the sensor data table 30 in which an association is made between sensor data and an obtainment time of the sensor data. Here, a configuration of the sensor data table 30 is illustrated in FIG. 15. The sensor data table 30 includes data items such as the time 31, biological data 32-1, biological data 32-2, biological data 32-3, the inclination sensor data 33, and the external sensor data 34. The time 31, the inclination sensor data 33, and the external sensor data 34 are identical to those of the second embodiment. The biological data 32-1, 32-2 and 32-3 are numerical values respectively indicated by a digital value obtained by performing an A/D conversion for the biological data obtained from the biological sensors 14-1, 14-2 and 14-3 at the time 31.

Additionally, the storage unit 16 stores correlation data indicating a relationship among the biological data 32-1, the biological data 32-2, the biological data 32-3, the ring inclination value 42, the external sensor data 34, and a correction value of the biological data. Similarly to the second embodiment, the correlation data has, for example, the form of a lookup table or a function expression.

The estimation unit 17 calculates a ring inclination value from the inclination sensor data 33, similarly to the first embodiment.

Then, the estimation unit 17 calculates a value of correction data on the basis of the biological data 32-1, 32-2 and 32-3 that are measured at the same time, the ring inclination value, and the value of the external sensor data 34. Then, the estimation unit 17 outputs the calculated correction data to the first transmission unit 18.

A method by which the estimation unit 17 calculates the value of correction data on the basis of values of a plurality of pieces of biological data 32 that are measured at the same time, the ring inclination value, and the value of the external sensor data 34 is described next.

Similarly to the second embodiment, the estimation unit 17 determines whether biological data is unsuitable data by judging whether the ring inclination value is equal to or smaller than a specified threshold value.

Then, the estimation unit 17 calculates correction data by using a plurality of pieces of biological data determined not to be unsuitable data. Here, the lookup table used in the third embodiment is a table implemented by adding data items of the plurality of pieces of biological data to the input information of the lookup table 40 in the second embodiment. Moreover, in the function expression, a plurality of pieces of biological data are additionally given to the input information of the function in the second embodiment. Assuming that the correction data, the plurality of pieces of biological data, the ring inclination value, and the external sensor data are respectively T', T1, T2, T3, θ and H, the function expression is

$$T' = f(T1, T2, T3, \theta, H)$$

A flow of operations of the nose ring sensing system according to the third embodiment is described next with reference to the flowchart illustrated in FIG. 8.

Initially, the control unit 15 obtains the biological data 32-1, 32-2, and 32-3 respectively from the biological sensors 14-1, 14-2 and 14-3, also obtains the inclination sensor data 33 from the inclination sensor 13, and further obtains the external sensor data 34 from the external sensor 24 (S52) . Then, the control unit 15 stores, in the sensor data table 30, the obtained biological data 32-1, 32-2, and 32-3, inclination sensor data 33, and external sensor data 34 in association with a measurement time.

Next, the estimation unit 17 obtains the inclination sensor data 33 from the sensor data table 30, and calculates a ring inclination value. Then, the estimation unit 17 determines whether the calculated ring inclination value is equal to or smaller than a specified threshold value (S53).

When the estimation unit 17 determines that the ring inclination value is equal to or smaller than the specified threshold value ("YES" in S53), the estimation unit 17 calculates correction data corresponding to the inclination sensor data 33 obtained in S52 (S54). The correction data is calculated on the basis of the lookup table or the function (LUT/Funct59). Then, the estimation unit 17 outputs, to the first transmission unit 18, the correction data calculated in S54, and the associated time 31. Next, the first transmission unit 18 transmits the input correction data and associated time 31 to the first reception unit 19 of the display terminal 12.

Upon receipt of the correction data and the associated time 31, the first reception unit 19 outputs the data and the time 31 to the display unit 20. The display unit 20 converts the received correction data and associated time 31 into a form that can be displayed, and displays the data and the time (S55). Then, the flow returns to the start of the process.

When the estimation unit 17 determines that the ring inclination value is larger than the specified threshold value ("NO" in S53), the flow returns to the start of the process.

(Fourth embodiment)

A configuration of a nose ring sensing system according to a fourth embodiment is identical to that of the third embodiment. The fourth embodiment is different from the third embodiment in that the biological sensor 14 closest to the nasal septum from among the plurality of biological sensors 14 is selected, and that data of the selected biological sensor 14 is corrected. Differences in the storage unit 16 and the estimation unit 17 from those of the third embodiment are described below.

The storage unit 16 stores the sensor data table 30 similarly to that of the third embodiment. Moreover, the storage unit 16 stores correlation data similarly to that of the second embodiment.

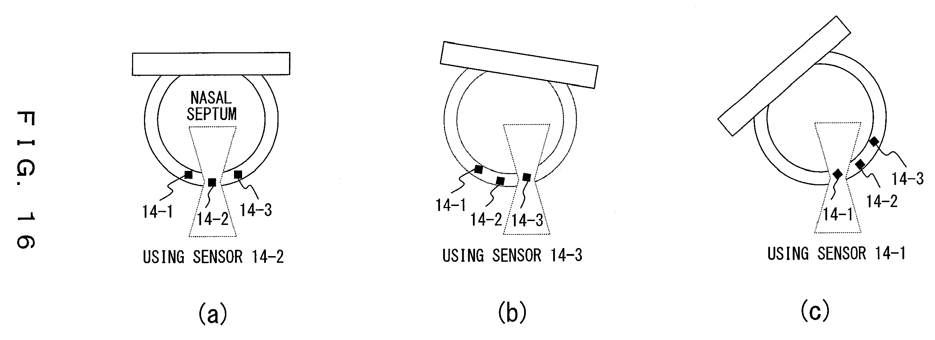

The estimation unit 17 selects the biological sensor 14 having the shortest distance from the nasal septum from among the plurality of biological sensors 14, and uses biological data measured by the selected biological sensor 14 to correct the data. FIG. 16 is an explanatory diagram for selecting the biological sensor 14 closest to the nasal septum from among the plurality of biological sensors 14. When the nose ring 11 does not become displaced as illustrated in FIG. 16(a), the biological sensor closest to the nasal septum is the biological sensor 14-2. In the meantime, when the nose ring 11 becomes displaced as illustrated FIGs. 16 (b) and 16(c), the biological sensor closest to the nasal septum is the biological sensor 14-1 or the biological sensor 14-3 in some cases. A method for identifying the biological sensor closest to the nasal septum from among the plurality of biological sensors is calculated, for example, on the basis of the ring inclination value, and positions in which the biological sensors are respectively placed.

The estimation unit 17 corrects the value of biological data measured by the selected biological sensor 14 on the basis of the ring inclination value calculated on the basis of inclination sensor data obtained at the same time as that of the biological data, and the value of the external sensor data. Then, the estimation unit 17 outputs the corrected biological data to the first transmission unit 18.

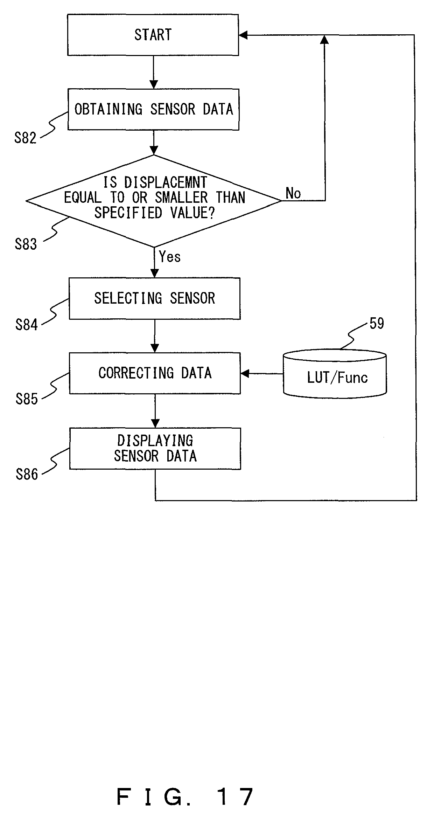

A flow of operations of the nose ring sensing system according to the fourth embodiment is described next. FIG. 17 illustrates the flow of operations of the nose ring sensing system according to the fourth embodiment.

Initially, the control unit 15 obtains biological data from the plurality of biological sensors 14, and also obtains inclination sensor data from the inclination sensor 13. Moreover, the control unit 15 obtains external sensor data from the external sensor 24 (S82). Then, the control unit 15 stores, in the sensor data table 30, the obtained biological data, inclination sensor data, and external sensor data in association with a measurement time.

Next, the estimation unit 17 obtains inclination sensor data from the sensor data table 30, and calculates a ring inclination value. Then, the estimation unit 17 determines whether the calculated ring inclination value is equal to or smaller than a specified threshold value (S83).

When the estimation unit 17 determines that the ring inclination value is equal to or smaller than the specified threshold value ("YES" in S83), the estimation unit 17 selects biological data of the biological sensor 14 having the closest distance from the nasal septum from among the biological data obtained in S82 (S84). Then, the estimation unit 17 corrects the selected biological data by using the ring inclination value and the external sensor data (S85). The correction data is calculated on the basis of the lookup table or the function (LUT/Func59). Then, the estimation unit 17 transmits the biological data corrected in S85 and the associated time to the display unit 20 via the first transmission unit 18.

Next, the display unit 20 converts the received biological data and associated time into a form that can be displayed, and displays the data and the time (S86). Then, the flow returns to the start of the process.

When the estimation unit 17 determines that the ring inclination value is larger than the specified threshold value in S83 ("NO" in S83), the flow returns to the start of the process.

(Fifth embodiment)

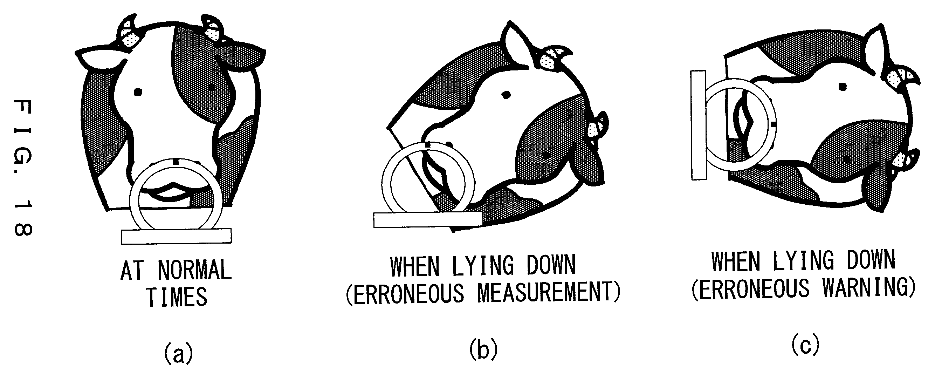

In the fifth embodiment, an inclination sensor is also attached to livestock, the inclination sensor of the nose ring 11 and that on the livestock are made mutually available, and data is corrected by taking into account the orientation of livestock so as to prevent erroneous detection when the livestock lies down. FIG. 18 is an explanatory diagram of erroneous detection or erroneous warnings being caused when livestock lies down. FIG. 18(a) illustrates the state of the nose ring 11 when livestock is standing up. In contrast, the nose ring 11 becomes displaced from the nasal septum in some cases even though the inclination of the nose ring 11 is small when the livestock lies down, as illustrated in FIG. 18 (b).

When a displacement of the nose ring 11 from the nasal septum is determined to have occurred on the basis of the inclination of the nose ring 11, the displacement of the nose ring 11 is determined to be small, leading to a possibility that biological data will not be corrected despite the displacement of the nose ring 11. Moreover, as illustrated in FIG. 18(c), the nose ring 11 does not become displaced from the nasal septum in some cases even though the inclination of the nose ring 11 is large. When the displacement of the nose ring 11 from the nasal septum is determined on the basis of the inclination of the nose ring 11 at this time, the displacement of the nose ring 11 is determined to be large, leading to a possibility that the detected biological data may be discarded.

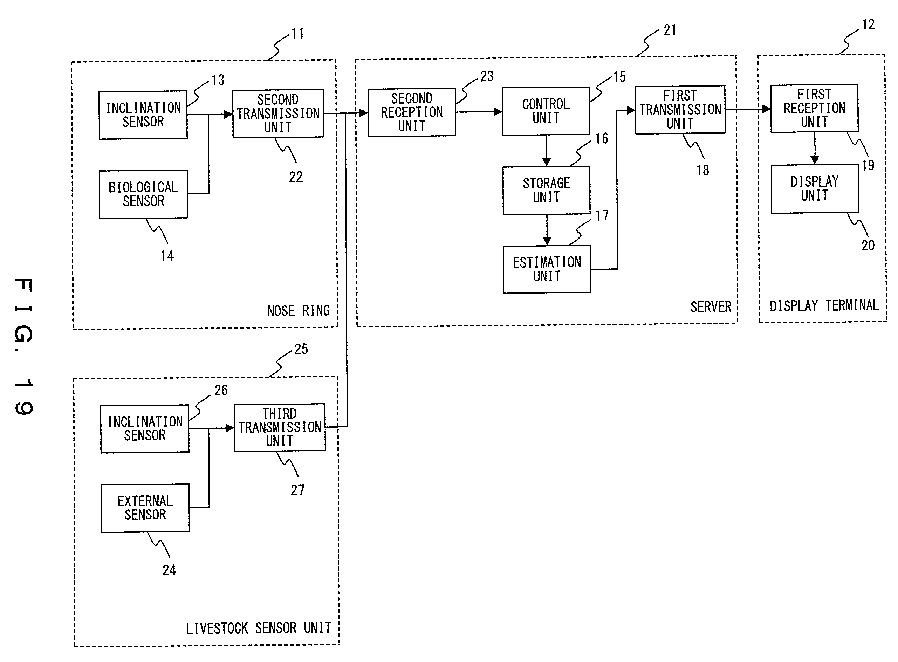

FIG. 19 illustrates a configuration of a nose ring sensing system according to the fifth embodiment. The fifth embodiment includes a livestock sensor unit 25 for measuring the inclination of the body of livestock in addition to the components of the first embodiment. The nose ring 11 and the display terminal 12 are identical to those described in the modification example 1. Differences in the control unit 15, the storage unit 16 and the estimation unit 17 from those of the first embodiment are described.

The livestock sensor unit 25 includes a body inclination sensor 26, the external sensor 24, and a third transmission unit 27. The livestock sensor unit 25 is placed, for example, in a certain position on the head of the livestock. The body inclination sensor 26 is one example of the inclination detection unit 5. The external sensor 24 is one example of the external information detection unit 4.

The body inclination sensor 26 is, for example, a three-axis acceleration sensor. The body inclination sensor 26 is placed so that a rotational axis of the inclination can become vertical to a surface of the nose ring 11, and is set so that data is transmitted to the server 21 at the same time as the nose ring 11.

The external sensor 24 is an external temperature sensor for measuring an external temperature.

The third transmission unit 27 transmits data measured by the body inclination sensor 26 and the external sensor 24 to the second reception unit 23 of the server 21 via a network.

The control unit 15 periodically obtains sensor data for a certain time period from the inclination sensor 13 and the biological sensor 14. Moreover, the control unit 15 simultaneously obtains inclination sensor data of livestock from the body inclination sensor 26 of the livestock sensor unit 25, and also obtains external sensor data from the external sensor 24. All pieces of sensor data obtained by the control unit 15 have an analog data format. The control unit 15 performs an A/D conversion for analog values of the obtained sensor data, and stores, in the storage unit 16, digital values of results of the conversion. Note that timings at which the control unit 15 obtains the sensor data from the inclination sensor 13, the biological sensor 14, and the body inclination sensor 26 are synchronized with one another.

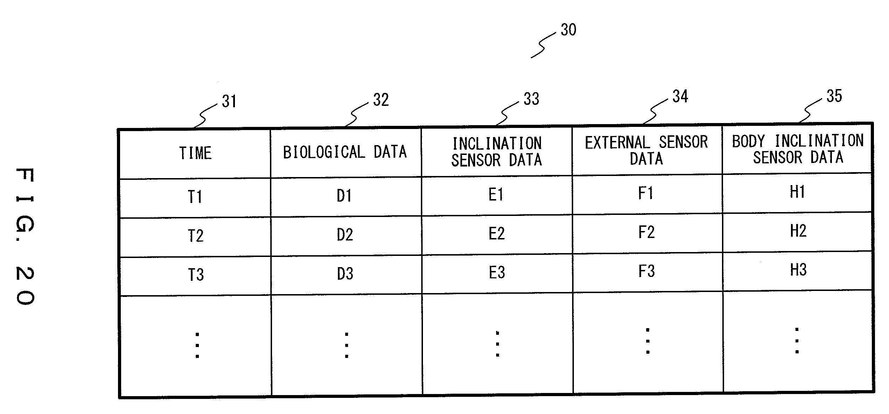

The storage unit 16 stores the sensor data table 30 in which an association is made between sensor data and a measurement time of the sensor data. The storage unit 16 receives, from the control unit 15, sensor data and a measurement time of the sensor data, and stores the received information in the sensor data table 30. Here, a structure of the sensor data table 30 is illustrated in FIG. 20. The sensor data table 30 includes data items such as the time 31, the biological data 32, the inclination sensor data 33, the external sensor data 34, and the body inclination sensor data 35. The time 31, the biological data 32 and the inclination sensor data 33 are identical to those of the first embodiment. The external sensor data 34 and the body inclination sensor data 35 are numerical values respectively indicated by a digital value of data measured by the external sensor 24 and the body inclination sensor 26 at the time 31.

Additionally, the storage unit 16 stores correlation data indicating a relationship among biological data, the inclination of the nose ring 11, the external sensor data, and a correction value of the biological data. The correlation data has, for example, the form of a lookup table or a function expression.

The estimation unit 17 calculates a ring inclination value from the inclination sensor data 33, similarly to the first embodiment.

Furthermore, the estimation unit 17 calculates a body inclination value, which is a value indicating an inclination of the body of livestock in the gravitational direction, from the body inclination sensor data 35 with a method similar to that for obtaining a ring inclination value from the inclination sensor data 33. Then, the estimation unit 17 calculates a difference between the calculated ring inclination value and the body inclination value. In the following explanation, the value of a thus-calculated difference is referred to as a correction ring inclination value.

The estimation unit 17 determines whether biological data is unsuitable data by judging whether the correction ring inclination value is equal to or smaller than a specified threshold value.

Then, the estimation unit 17 corrects the biological data determined not to be unsuitable data. Here, the lookup table used in the fifth embodiment includes, as input information, data items such as biological data, a correction ring inclination value, and external sensor data. Moreover, the function expression has a form such that biological data, a correction ring inclination value, and external sensor data are given as input information of the function as follows. Assuming that the correction data, the biological data, the correction ring inclination value, and the external sensor data are respectively T', T, θ, and H, the functional expression is

$$T' = f(T, \theta, H)$$

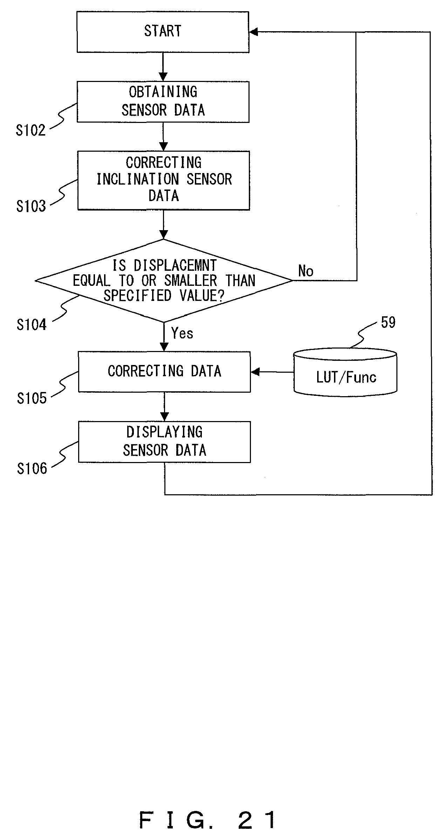

A flow of operations of the nose ring sensing system according to the fifth embodiment is described next. FIG. 21 is a flowchart illustrating operations of the nose ring sensing system according to the fifth embodiment.

Initially, the control unit 15 obtains biological data from the biological sensor 14, and also obtains inclination sensor data from the inclination sensor 13. Moreover, the control unit 15 obtains external sensor data from the external sensor 24, and also obtains body inclination sensor data from the body inclination sensor 26 (S102). Then, the control unit 15 stores, in the sensor data table 30, the obtained biological data, inclination sensor data, external sensor data, and body inclination sensor data in association with a measurement time.

Next, the estimation unit 17 obtains the inclination sensor data and the body inclination sensor data from the sensor data table 30, and calculates a correction ring inclination value (S103). Then, the estimation unit 17 determines whether the calculated correction ring inclination value is equal to or smaller than a specified threshold value (S104).

When the estimation unit 17 determines that the correction ring inclination value is equal to or smaller than the specified threshold value ("YES" in S104), the estimation unit 17 calculates correction data by using a plurality of pieces of biological data, the correction ring inclination value, and the external sensor data (S105). The correction data is calculated on the basis of the lookup table, or the function (LUT/Func59). Then, the estimation unit 17 transmits the correction data calculated in S105 and a time corresponding to the correction data to the display unit 20 via the first transmission unit 18.

Next, the display unit 20 converts the received correction data and time corresponding to the correction data into a form that can be displayed, and displays the data and the time (S106). Then, the flow returns to the start of the process.

When the estimation unit 17 determines that the ring inclination value is larger than the specified threshold value in S104 ("NO" in S104), the flow returns to the start of the process.

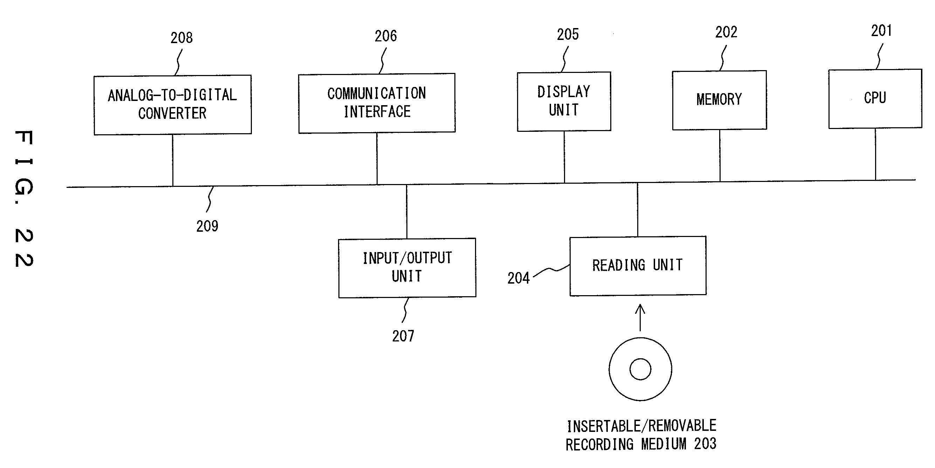

FIG. 22 illustrates one example of a hardware configuration of the nose ring 11, the server 21, and the display terminal 12 according to the embodiments. The nose ring 11, the server 21, and the display terminal 12 include some or all of a CPU 201, a memory 202, a reading unit 204, a display device 205, a communication interface 206, an input/output device 207, and an analog-to-digital converter 208 as illustrated in FIG. 22. The CPU 201, the memory 202, the reading unit 204, the display device 205, the communication interface 206, the input/output device 207, and the analog-to-digital converter 208 are interconnected, for example, via a bus 209.

Specifically, the nose ring 11 in the first to the fourth embodiments includes the CPU 201, the memory 202, the communication interface 206, the input/output device 207, and the analog-to-digital converter 208. The nose ring 11 in the fifth embodiment and the modification example includes the communication interface 206, and the input/output device 207. The server 21 in the fifth embodiment and the modification example includes the CPU 201, the memory 202, the reading unit 204, the communication interface 206, the input/output device 207, and the analog-to-digital converter 208. The livestock sensor unit 25 in the fifth embodiment includes the communication interface 206, and the input/output device 207. The display terminal 12 in the first to the fifth embodiments includes the display device 205, and the communication interface 206.

The CPU (Central Processing Unit) 201 executes, by using the memory 202, a program that describes the steps of the above described flowcharts. The CPU 201 offers some or all of the functions of the control unit 15 and the estimation unit 17.

The memory 202 is, for example, a semiconductor memory, and is configured by including a RAM (Random Access Memory) area, and a ROM (Read Only Memory) area. The memory 202 offers some or all of the functions of the storage unit 16. The memory 202 may include a semiconductor memory such as a flash memory or the like, or may include a hard disk.

The reading unit 204 accesses an insertable/removable recording medium 203 in accordance with an instruction of the CPU 201. The insertable/removable recording medium 203 is implemented, for example, with a semiconductor device (a USB memory or the like), a medium (a magnetic disk or the like) to and from which information is input and output with a magnetic action, a medium (a CD-ROM, a DVD or the like) to and from which information is input and output with an optical action, or other media.

The display device 205 displays biological data. The display device 205 offers some or all of the functions of the display unit 20.

The communication interface 206 transmits and receives data via a network. The communication interface 206 offers some or all of the functions of the first transmission unit 18, the first reception unit 19, the second transmission unit 22, the second reception unit 23, and the third transmission unit 27.

The input/output device 207 is equivalent to, for example, a device that accepts an instruction from a user. The input/output device 207 is used by a user to set the lookup table 40. Moreover, the input/output device 207 is used to input sensor data from the inclination sensor 13, the biological sensor 14, the external sensor 24, and the body inclination sensor 25.

The analog-to-digital converter 208 converts an analog value of sensor data into a digital value. The analog-to-digital converter 208 offers some or all of the functions of the control unit 15.

An information processing program for implementing the embodiments is provided to the nose ring 11 or the server 21, for example, in the following forms.

(1) Preinstalled in the memory 202.
(2) Provided by the insertable/removable recording medium 203.
(3) Provided via a network.

The embodiments are not limited to the above described ones, and can take various configurations or embodiments within a scope that does not depart from the gist of the embodiments.

The lookup table 40 or the function expression of correlation data used for correcting sensor data may be set or set to be corrected for one or more of the type, the sex, the age, the nose size, and the nasal septum thickness of livestock. For example, one or more of the entries such as the type, the sex, the age, the nose size, and the nasal septum thickness of livestock may be further included as input information of the lookup table 40 or the function expression. Additionally, the server 21 may be implemented by part of a system that configures a cloud.

Additionally, the timings at which the control unit 15 obtains sensor data from the inclination sensor 13, the biological sensor 14, and the body inclination sensor 26 are synchronized with one another in the first to the fifth embodiments. However, the timings are not limited to the above described ones. Namely, to obtain sensor data measured at the same time from a plurality of sensors, for example, the plurality of sensors may respectively have timers synchronized with one another among the plurality of sensors or between the plurality of sensors and a server, and the sensor data may be transmitted to the control unit 15 at specified time intervals. Moreover, the timers may be synchronized with one another with various methods such as a method using an NTP (Network Time Protocol) or the like from among the plurality of sensors, or between the plurality of sensors and a server via a network.

Furthermore, in the second to the fourth embodiments, some of the functions of the nose ring 11 may be executed by the server 21 as in the modification example of the first embodiment. Moreover, the functions of the server 21 in the fifth embodiment may be executed by the nose ring 11.

**Claims**

1. A biological sensing system, comprising:

   a biological information detection unit that is placed in a first specified portion of a living thing, and is configured to detect biological information of the living thing;
   a position displacement detection unit configured to detect an amount of a displacement of a position of the biological information detection unit from the first portion when the biological information is detected; and

an estimation unit configured to estimate optimum biological information from the biological information detected by the biological information detection unit in accordance with the amount of the displacement.

2. The biological sensing system according to claim 1, wherein
the estimation unit selects or abandons the biological information in accordance with the amount of the displacement, and estimates the selected biological information as the optimum biological information.

3. The biological sensing system according to claim 1 or 2, wherein
the position displacement detection unit detects a change in the acceleration caused by gravity, and detect the amount of the displacement on the basis of the change in the acceleration of gravity, and when the amount of the displacement is equal to or smaller than a specified threshold value, the estimation unit estimates the biological information as the optimum biological information.

4. The biological sensing system according to any one of claims 1 to 3, wherein
the estimation unit corrects the biological information in accordance with the amount of the displacement, and estimates the corrected biological information as the optimum biological information.

5. The biological sensing system according to any one of claims 1 to 4, further comprising
a plurality of the biological information detection units, wherein
the estimation unit selects one piece of biological information in accordance with the amount of the displacement from among the plurality of pieces of biological information detected by the plurality of biological information detection units, and estimates the selected biological information as the optimum biological information.

6. The biological sensing system according to claim 5, wherein
the estimation unit corrects the selected one piece of biological information in accordance with the amount of the displacement, and estimates the corrected biological information as the optimum biological information.

7. The biological sensing system according to any one of claims 1 to 3, comprising
a plurality of biological information detection units, wherein
the estimation unit estimates the optimum biological information on the basis of the plurality of pieces of biological information detected by the plurality of biological information detection units, and the amount of the displacement.

8. The biological sensing system according to any one of claims 1 to 3, further comprising
an external information detection unit configured to detect external information about a periphery of the living thing when the biological information is detected, wherein
the estimation unit corrects the biological information detected by the biological information detection unit in accordance with the external information detected by the external information detection unit, and the amount of the displacement, and estimates the corrected biological information as the optimum biological information.

9. The biological sensing system according to claim 7, further comprising
an external information detection unit configured to detect external information about a periphery of the living thing when the biological information is detected, wherein
the estimation unit estimates the biological information of the living thing in accordance with the external information detected by the external information detection unit, the amount of the displacement, and the plurality of pieces of biological information detected by the plurality of biological information detection units.

10. The biological sensing system according to any one of claims 1 to 9, further comprising
an inclination detection unit that is placed in a second portion of the living thing, and is configured to detect an inclination of the second portion in a gravitational direction, wherein
the estimation unit corrects the amount of the displacement on the basis of the inclination of the second portion in the gravitational direction.

11. The biological sensing system according to any one of claims 1 to 10, wherein
the estimation unit corrects the biological information detected by the biological information detection unit in accordance with a type, a sex, an age, a nose size, and a nasal septum thickness of the living thing.

12. A biological sensing method, comprising:

obtaining biological information detected by a biological information detection unit that is placed in a specified portion of a living thing, and is configured to detect the biological information of the living thing;
obtaining an amount of a displacement detected by a position displacement detection unit that detects the amount of the displacement of a position of the biological information detection unit from the specified portion when the biological information is detected; and

estimating optimum biological information from the obtained biological information in accordance with the amount of the displacement.

13. A biological sensing program for causing a processor to execute a process comprising:

obtaining biological information detected by a biological information detection unit that is placed in a specified portion of a living thing, and is configured to detect the biological information of the living thing;

obtaining an amount of a displacement detected by a position displacement detection unit that detects the amount of the displacement of a position of the biological information detection unit from the specified portion when the biological information is detected; and

estimating optimum biological information from the obtained biological information in accordance with the amount of the displacement.

EP 3 064 060 A1

F I G. 1

F I G. 2

28

29

F I G. 3

30

| TIME | BIOLOGICAL DATA | INCLINATION SENSOR DATA |
|------|-----------------|-------------------------|
| 31 | 32 | 33 |
| T1 | D1 | E1 |
| T2 | D2 | E2 |
| T3 | D3 | E3 |
| ⋮ | ⋮ | ⋮ |

F I G. 4

F I G. 5

F I G. 6

40

| | 41 | 42 | 43 |
|---|---|---|---|
| | INPUT | | OUTPUT |
| | BIOLOGICAL DATA | RING INCLINATION VALUE | CORRECTION DATA |
| | D1 | Θ1 | D'11 |
| | D1 | Θ2 | D'12 |
| | ⋮ | ⋮ | ⋮ |
| | D1 | Θx | D'1x |
| | D2 | Θ1 | D'21 |
| | ⋮ | ⋮ | ⋮ |

F I G. 7

START

OBTAINING SENSOR
DATA

S52

IS DISPLACEMENT
EQUAL TO OR SMALLER THAN
SPECIFIED VALUE?

No

S53

Yes

CORRECTING DATA

LUT/Func

59

S54

DISPLAYING SENSOR
DATA

S55

F I G. 8

F I G. 9

F I G. 1 0

| TIME | BIOLOGICAL DATA | INCLINATION SENSOR DATA | EXTERNAL SENSOR DATA |
|------|-----------------|-------------------------|----------------------|
| T1 | D1 | E1 | F1 |
| T2 | D2 | E2 | F2 |
| T3 | D3 | E3 | F3 |
| ⋮ | ⋮ | ⋮ | ⋮ |

FIG. 11

40

41      42      44      43

| INPUT | | | OUTPUT |
|---|---|---|---|
| BIOLOGICAL DATA | RING INCLINATION VALUE | EXTERNAL SENSOR DATA | CORRECTION DATA |
| D1 | Θ1 | F1 | D'111 |
| D1 | Θ1 | F2 | D'112 |
| ⋮ | ⋮ | ⋮ | ⋮ |
| D1 | Θ1 | Fy | D'11y |
| D2 | Θ2 | F1 | D'121 |
| D2 | Θ2 | F2 | D'122 |
| ⋮ | ⋮ | ⋮ | ⋮ |
| D2 | Θx | Fy | D'1xy |
| ⋮ | ⋮ | ⋮ | ⋮ |
| Dz | Θ1 | F1 | D'z11 |
| ⋮ | ⋮ | ⋮ | ⋮ |
| Dz | Θx | Fy | D'zxy |

F I G. 1 2

F I G. 1 3

14-1  14-2  14-3

F I G. 1 4

# F I G. 1 5

| TIME | BIOLOGICAL DATA | BIOLOGICAL DATA | BIOLOGICAL DATA | INCLINATION SENSOR DATA | EXTERNAL SENSOR DATA |
|------|-----------------|-----------------|-----------------|-------------------------|----------------------|
| T1 | D11 | D21 | D31 | E1 | F1 |
| T2 | D12 | D22 | D32 | E2 | F2 |
| T3 | D13 | D23 | D33 | E3 | F3 |
| ⋮ | ⋮ | ⋮ | ⋮ | ⋮ | ⋮ |

(a) USING SENSOR 14-2

NASAL SEPTUM

14-1 14-2 14-3

(b) USING SENSOR 14-3

14-1 14-2 14-3

(c) USING SENSOR 14-1

14-3 14-2 14-1

F I G. 1 6

START

↓

OBTAINING SENSOR DATA

S82

↓

IS DISPLACEMNT
EQUAL TO OR SMALLER THAN
SPECIFIED VALUE?

S83

No →

Yes ↓

SELECTING SENSOR

S84

↓

CORRECTING DATA ← LUT/Func 59

S85

↓

DISPLAYING
SENSOR DATA

S86

F I G. 1 7

F I G. 1 8

AT NORMAL
TIMES

(a)

WHEN LYING DOWN
(ERRONEOUS MEASUREMENT)

(b)

WHEN LYING DOWN
(ERRONEOUS WARNING)

(c)

F I G. 19

F I G. 2 0

| TIME | BIOLOGICAL DATA | INCLINATION SENSOR DATA | EXTERNAL SENSOR DATA | BODY INCLINATION SENSOR DATA |
|------|-----------------|-------------------------|----------------------|------------------------------|
| T1 | D1 | E1 | F1 | H1 |
| T2 | D2 | E2 | F2 | H2 |
| T3 | D3 | E3 | F3 | H3 |
| ⋮ | ⋮ | ⋮ | ⋮ | ⋮ |

```
                          ┌─────────────────┐
                          │      START      │◄──────────────┐
                          └────────┬────────┘               │
                                   │                        │
                          ┌────────▼────────┐               │
                          │    OBTAINING    │               │
                          │   SENSOR DATA   │               │
        S102              └────────┬────────┘               │
                                   │                        │
                          ┌────────▼────────┐               │
                          │    CORRECTING   │               │
                          │ INCLINATION SENSOR │            │
                          │      DATA       │               │
        S103              └────────┬────────┘               │
                                   │                        │
                                   ▼                        │
                        ◄───────────────────────►           │
                          IS DISPLACEMNT              No     │
                     EQUAL TO OR SMALLER THAN ─────────────┘
                          SPECIFIED VALUE?
                        ◄───────────────────────►
        S104                       │
                                   │ Yes                    ┌────────┐ 59
                          ┌────────▼────────┐               │
                          │  CORRECTING DATA │◄───┌─────────────┐
                          └────────┬────────┘     │  LUT/Func   │
        S105                       │              └─────────────┘
                          ┌────────▼────────┐
                          │    DISPLAYING   │
                          │   SENSOR DATA   │
        S106              └────────┬────────┘
                                   │
                                   └────────────────────────┘
```

F I G. 2 1

# F I G. 2 2

| 208 ANALOG-TO-DIGITAL CONVERTER | 206 COMMUNICATION INTERFACE | 205 DISPLAY UNIT | 202 MEMORY | 201 CPU |

209

207 INPUT/OUTPUT UNIT

204 READING UNIT

INSERTABLE/REMOVABLE RECORDING MEDIUM 203

## INTERNATIONAL SEARCH REPORT

| | International application No. |
|---|---|
| | PCT/JP2013/079462 |

**A. CLASSIFICATION OF SUBJECT MATTER**
*A01K67/00*(2006.01)i, *A01K15/00*(2006.01)i

According to International Patent Classification (IPC) or to both national classification and IPC

**B. FIELDS SEARCHED**

Minimum documentation searched (classification system followed by classification symbols)
A01K67/00, A01K15/00, A01K11/00, A61D13/00, G01K1/00-19/00, A61B5/00-5/22

Documentation searched other than minimum documentation to the extent that such documents are included in the fields searched
Jitsuyo Shinan Koho          1922-1996     Jitsuyo Shinan Toroku Koho     1996-2014
Kokai Jitsuyo Shinan Koho     1971-2014     Toroku Jitsuyo Shinan Koho     1994-2014

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2013-48581 A  (Fujitsu Ltd.),<br>14 March 2013 (14.03.2013),<br>entire text; all drawings<br>(Family: none) | 1-13 |
| A | JP 2010-68268 A  (Wacom-IT Co., Ltd.),<br>25 March 2010 (25.03.2010),<br>entire text; all drawings<br>(Family: none) | 1-13 |

[X]  Further documents are listed in the continuation of Box C.          [ ]  See patent family annex.

| | |
|---|---|
| *      Special categories of cited documents:<br>"A"    document defining the general state of the art which is not considered    to be of particular relevance<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| 23 January, 2014 (23.01.14) | 04 February, 2014 (04.02.14) |

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2013/079462

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-541866 A (Maasland N.V.), 10 December 2002 (10.12.2002), entire text; all drawings & EP 1089617 A          & WO 2000/064245 A1 & DE 60033899 D         & NL 1011884 C & AU 3464300 A          & CA 2334800 A & AT 356547 T           & DK 1089617 T & NL 1011884 C2 | 1-13 |
| A | JP 2011-185820 A (Seiko Epson Corp.), 22 September 2011 (22.09.2011), entire text; all drawings & US 2011/0224936 A1     & CN 102221415 A | 1-13 |
| A | US 2010/0036277 A1 (John Austin), 11 February 2010 (11.02.2010), entire text; all drawings & GB 2453694 A            & WO 2008/003139 A1 & AU 2007271732 A | 1-13 |
| A | US 2005/0209526 A1 (Herbert A. Ingley III), 22 September 2005 (22.09.2005), entire text; all drawings & WO 2005/058192 A2 | 1-13 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002541866 A